# EUROPEAN PATENT SPECIFICATION

(11) **EP 1 651 165 B1**
(45) Date of publication and mention of the grant of the patent: **03.06.2009**
(21) Application number: 04763677.4
(22) Date of filing: 30.07.2004
(51) Int. Cl.: A61K 6/00, A61K 6/087

(54) **DENTAL ROOT CANAL SEALING COMPOSITION**
ZAHNWURZELKANAL-VERSCHLUSSZUSAMMENSETZUNG
COMPOSITION DE SCELLEMENT DU CANAL D'UNE RACINE DENTAIRE

(30) Priority: 31.07.2003 EP 03017391; 10.03.2004 US 551348 P
(43) Date of publication of application: 03.05.2006
(73) Proprietor: DENTSPLY DETREY GmbH, 78467 Konstanz (DE)
(72) Inventor: KLEE, Joachim, E., 78315 Radolfzell (DE)
(74) Representative: Hartz, Nikolai
(86) International application number: PCT/EP2004/008599
(87) International publication number: WO 2005/013922

(56) References cited:
- EP-A- 0 673 637
- WO-A-02/13767
- US-A1- 2003 045 604

## Description

### Field of the invention

The present invention relates to a dental root canal sealing composition curable by addition polymerisation in the absence of a polymerisation catalyst.The dental root canal sealing composition of the invention is provided in the form of a two-component composition.

### Background art

Dental root canal sealing compositions are frequently applied into the root canal through a canal of a needle. Due to the small dimensions of the needle canal, the compositions are required to have a low viscosity. Alternatively, dental root canal sealing compositions are applied by using lentulos or gutta percha tips. Accordingly, the viscosity must be low so that thin films may be formed. Independent from the application technique, the viscosity of the material must be low enough so that the composition may enter into dentine canals in the root canal.

The application of dental root canal sealing compositions is checked by using X-ray procedures. Due to the requirement for radioopacity, the compositions are required to contain a substantial amount of a radioopaque filler.

Dental root canal sealing compositions are known from WO 02/13767 disclosing in the application examples a two-component paste/paste system. The two-component paste/paste system is based on addition polymerisation of equimolar amounts of low-molecular diamines and low-molecular diacrylates optionally in the presence of a reactive diluent for ajusting the viscosity of the composition.

However, the presence of low molecular amines in the dental root canal sealing composition leads to severe drawbacks. Cytotoxic effects are frequently observed due to leaching of such amines from the root canal. Moreover, the cured compositions of WO 02/13767 show a considerable solubility whereby the cytotoxicity problem is aggravated and further application problems are created. Finally, the high vapor pressure of low molecular amines and the high penetration rate through plastic packaging render the compositions of WO 02/13767 problematic for industrial application.

Polyaminoesters specifically disclosed in WO 02/13767 are highly viscous and require the use of a substantial amount of reactive diluent in order to decrease the viscosity. However, reactive diluents cannot be polymerised by addition polymerisation, but require the presence of a polymerisation initiator.

WO 02/13767 does not disclose a di-or polyfunctional acrylate compound or a di- or polyfunctional maleimide compound which is capable of undergoing polyaddition.

It is the problem of the present invention to provide a dental root canal sealing composition having a low viscosity, low cytotoxicity, and low solubility while having excellent mechanical properties such as low shrinkage and flexibility and which do not give rise to handling problems during manufacture and application.

### Summary of the invention

This problem is solved according to the claims. The present invention provides a dental root canal sealing composition curable in the absence of a polymerisation initiator, which comprises
(i) an aminoterminated prepolymer having a viscosity at 23°C of less than 100 Pa*s, which is obtainable by reacting
   (a) one mole of a compound of the following formula (I) 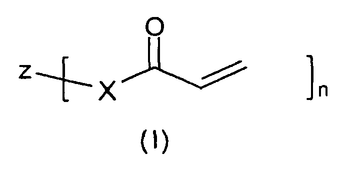 wherein
      X represents a nitrogen or an oxygen atom;
      Z represents an n-valent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups; and
      n represents an integer of from 2 to 6; and
   (b) at least n moles of one or more compounds either of the following formula (II) 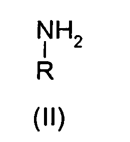
   wherein
   R represents
   a C₁₋₆ alkyl or a C₃₋₁₄ cycloalkyl group, which may be substituted by one or more (up to six) members of the group selected from a C₁₋₄ alkyl group, C₁₋₄ alkoxy group, a phenyl group, and a hydroxy group,or
   of formula (II) in combination with one or more di- or polyamine compounds.
(ii) a di-or polyfunctional acrylate compound or a di- or polyfunctional maleimide compound which is capable of undergoing polyaddition with the aminoterminated prepolymer (i);
(iii) 40 to 85 wt.-% of a filler for providing a minimum radioopacity of at least 3mm/mm AI;
said compositon being in the form of a two-component composition wherein a first component contains the amino terminated prepolymer (i) and optionally filler (iii) and a second component contains the compound (ii) capable of undergoing polyaddition with the aminoterminated prepolymer (i) and optionally filler (iii).

In the formulae, X represents a nitrogen or an oxygen atom. In the formulae, Z is an n-valent saturated aliphatic C₂₋₁₆ hydrocarbon group or a divalent saturated cycloaliphatic C₃₋₆ hydrocarbon group, which groups may be based on linear or branched alkylene groups having 2 to 16 carbon atoms, preferably 4 to 10 carbon atoms, or cycloalkylene groups having 3 to 6 carbon atoms, preferably 4 to 6 carbon atoms. Z may be divalent (n=2), trivalent (n=3), tetravalent (n=4), pentavalent (n=5), or hexavalent (n=6). Preferable Z is divalent or trivalent. The hydrocarbon group may be substituted by one or more C₁₋₄ alkyl groups. Specific examples of the alkyl groups are methyl, ethyl, n-propyl, i-propyl, n-butyl, i-butyl or tert.-butyl. The hydrocarbon group may contain 1 to 6 oxygen atoms in the carbon chain connecting the n acrylate substituents or in a side chain. Preferably the aliphatic or cycloaliphatic C₂₋₁₆ hydrocarbon group is highly flexible due to the presence of ether bonds and the absence of bulky groups. In a preferred embodiment, Z is a divalent group based on a straight chain alkyl group which may contain ether bonds. Specifically, Z may be a C₂₋₆ alkylene group.

In formula (II), R represents a C₁₋₆ alkyl or a C₃₋₁₄ cycloalkyl group. Examples for a C₁₋₆ alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. Examples of the C₃₋₁₄ cycloalkyl group can include those having 3 to 14 carbon atoms, for example, cyclopropyl, cyclobutyl, cyclopentyl and cyclohexyl. The C₁₋₆ alkyl group and the C₃₋₁₄ cycloalkyl group may optionally be substituted by one or more members (up to six) of the group selected from a C₁₋₄ alkyl group, C₁₋₄ alkoxy group, a phenyl group, and a hydroxy group. Examples for a C₁₋₄ alkyl group can include linear or branched alkyl groups having 1 to 4 carbon atoms, for example, methyl, ethyl, n-propyl, isopropyl, n-butyl, isobutyl, sec-butyl, tert-butyl. Examples for an C₁₋₄ alkoxy group can include linear or branched alkoxy groups having 1 to 4 carbon atoms, for example, methoxy, ethoxy, n-propoxy, isopropoxy, n-butoxy, isobutoxy, sec-butoxy, and tert-butoxy.

In the preparation of the prepolymer, the compound of formula (II) may be optionally used in combination with with one or more di- or polyamine compounds. The di- or polyamine compound may be used to replace up to n/1.5 moles, preferably n/20 to n/2 moles of the compound of formula (II) used in the reaction for preparing the prepolymer, wherein n is as defined above. The amount of the component used in combination with the amine of formula (II) must be chosen such that the viscosity of the prepolymer does not exceed 100 Pa*s, preferably 80 Pa*s, more preferably 20 Pa*s.

### Description of the preferred embodiments

In a preferred embodiment the dental root canal sealing composition contains a prepolymer of the following formula (III) 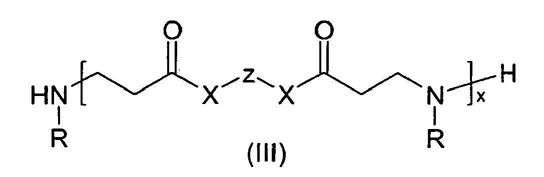 wherein
- X: represents an oxygen or nitrogen atom;
- Z: represents an n-valent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups; and
- R: represent a C₁₋₄ alkyl or a C₃₋₁₄ cycloalkyl group, which may be substituted by a C₁₋₄ alkyl group, C₁₋₄ alkoxy group, a phenyl group, or a hydroxy group; and
- x: is an integer of from 1 to 8;

(ii) a di-or polyfunctional acrylate compound or a di- or polyfunctional maleimide compound which is capable of undergoing polyaddition with the aminoterminated prepolymer (i);
(iii) 40 to 85 wt.-% of a filler for providing a minimum radioopacity of at least 3mm/mm Al.

The dental root canal sealing compositon is in the form of a two-component composition wherein a first component contains the amino terminated prepolymer (i) and optionally filler (iii) and a second component contains the compound (ii) capable of undergoing polyaddition with the aminoterminated prepolymer (i) and optionally filler (iii). At least one of the components contains filler.

The dental root canal sealing composition of the invention contains 40 to 85 wt.-% of a filler for providing a minimum radioopacity of the cured composition of at least 3mm/mm Al. The filler contains La₂O₃, ZrO₂, BiPO₄, CaWO₄, BaWO₄, SrF₂, Bi₂O₃. The radioopacity of the cured composition of the invention is at least 3mm/mm Al, preferably at least 5 to 7 mm/mm Al, and most preferably at least 7 mm/mm Al.

Preferably, the dental root canal sealing composition of the invention does not contain a diluent, in particular a reactive diluent, having a viscosity which is lower than the viscosity of the prepolymer of the invention. Moreover, the dental root canal sealing composition does not need to contain a polymerisation initiator. In a preferred embodiment, the dental root canal sealing composition consists essentially of components (i) to (iii). A dental root canal sealing composition consisting essentially of components (i) to (iii) may contain common additives used in the dental field such as colorants, antibiotic agents and ion releasing agents, in a total amount of not more than 25 wt.-%, preferably not more than 10 wt.% of the composition.

A preferred embodiment of the dental root canal sealing composition of the invention contains 40 to 85 wt.-% of a filler and 15 to 60 wt.-% of the aminoterminated prepolymer and the compound capable of undergoing polyaddition with the aminoterminated prepolymer. The aminoterminated prepolymer used in the present invention is usually a mixture of oligomers. Accordingly, the amount of the aminoterminated prepolymer and the compound capable of undergoing polyaddition with the aminoterminated prepolymer is calculated based on the mixture of oligomers.

The dental root canal sealing composition of the present invention is a two component composition which is mixed prior to use.The two component composition is preferably a powder/liquid system, a powder/paste system, a paste/paste system or a liquid/paste system. The paste/paste system or a liquid/paste system may be applied by an applicator wherein both components are mixed by a static mixer.

The present invention is based on the recognition that the Michael addition of specific diamines to specific di- and oligoacrylate compounds provides prepolymers having low viscosity while at the same time eliminating the problems associated with the presence of low molecular amines. Surprisingly, the reaction kinetics of the Michael addition of diamines with acrylates differs from the reaction kinetics of other addition reactions of amines so that the reaction products are not crosslinked or high molecular highly viscous materials, but uncrosslinked prepolymers having low viscosity. It appears that the difference of the reaction rates between primary and secondary amines with acrylate compounds is the basis for the possibility of obtaining the compositions of the present invention. Based on the specific reaction kinetics of primary amines in the present invention, the content of residual primary amines in the prepolymer or in the dental root canal sealing composition of the present invention is substantially reduced as compared to systems using functional end groups showing the same reactivity towards primary and secondary amines, cf. J. Klee, H.-H. Hörhold, J. Raddatz; Acta Polymerica, 41 (1990) 557-560; "Telechele Prepolymere aus DGEBA und disekundären Diaminen. Unvernetzte Epoxid-Amin-Additionspolymere, 29."; J.E. Klee; Acta Polym. 45 (1994) 73-82; "Telechelic prepolymers and macromonomers by step growth processes" (39); J. E. Klee, R.-E. Grützner, H.-H. Hörhold; Macromol. Chem. Phys. 197 (1996) 2305-2323; Linear aryalamine/ Bis-2,2-[4-(2,3-epoxypropoxy)-phenyl]-propane addition polymers - synthesis and properteis, Uncrosslinked epoxide-amine addition polymers, 44." Primary amines react much faster as compared to secondary amines, in particular in the reaction with di- or polyfunctional acrylates or acrylamides, or bis- or polymaleimides.

The prepolymer contained in the dental root canal sealing composition of the present invention has a viscosity at 23°C of less than 100 Pa*s. Preferably, the viscosity of the prepolymer is in the range of from 1 to 80 Pa*s, more preferably from 1 to 20 Pa*s. If the viscosity is too high, then it will be difficult to apply the composition through the canal of a needle. If the viscosity is too low, then it will be difficult handle the composition.

The dental root canal sealing composition of the present invention is curable in the absence of a polymerisation initiator. The curing mechanism is based on an addition reaction of an addition reaction between the aminoterminated prepolymer (i) and a compound capable of undergoing polyaddition with the aminoterminated prepolymer (i). The compound capable of undergoing polyaddition with the aminoterminated prepolymer (i) is a di- or polyfunctional acylate, or di- or polyfunctional maleimide. Di-or polyfunctional acrylates and di- or polyfunctional maleimides are preferred with regard to the selectivity in the reaction with primary and secondary amino groups. The compound capable of undergoing polyaddition with the aminoterminated prepolymer (i) may be the same or different as the compound as defined by formula (I) wherein X and Z are as defined above and n represents an integer of from 2 to 6.

The use of the specific amino terminated prepolymers eliminates or at least strongly reduces the problems associated with low molecular amines. Moreover, it is surprising that the use of the specific prepolymers provides a dental root canal sealing composition which has a low viscosity. For this purpose, it is essential that rigid moieties in the prepolymers are avoided.

The compositions of the pesent invention may be applied to a root canal by using conventional techniques. Specifically, the compositions of the present invention may be applied via the canal of a syringe into the root canal. Moreover, the compositions of the present invention may also be used for the manufacture of prefabricated root canal cones. If cones made of the compositions of the invention are used in combination with the respective dental root canal sealing composition of the invention, compatibility of the cones with the sealing composition can be guaranteed whereby a tight seal may be obtained. The cured product obtained with the composition according to the invention has superior mechanical properties, in particular with regard to flexibility, which is essential for the application as a root canal sealing composition.

Now, the general process for the preparation of the an amino terminated prepolymer is described. The amino terminated prepolymer may be obtained by reacting
(a) one mole of a compound of the following formula (I) 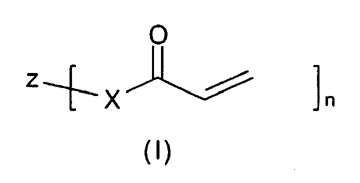 wherein
   X and Z are as defined above and
   n represents an integer of from 2 to 4;
(b) at least n moles of one or more compounds either of the following formula (II) 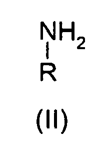 wherein
   R is as defined above, or of formula (II) in combination with one or more di- or polyamine compound.

The reaction may be carried out in the absence of a solvent or in the presence of a suitable solvent. The temperature of the reaction is preferably in the range of from 10 °C to 150 °C, more preferably in the range of 20 °C to 80 °C. The reaction time depends on the temperature and the reactivity of the reaction system and is usually in the range of from hours to several days. The termination of the reaction may be checked by conventional methods such as an IR spectrum whereby the end of the reaction is reached wen all acrylic carbon-carbon double bonds have disappeared. In case the reaction is carried out in the absence of a solvent, the prepolymers obtained by the reaction of compounds (I) and (II) may be used as such without further work-up of the reaction mixture. In the preparation of the prepolymer, the compound of formula (II) may be used in combination with one or more di- or polyamine compounds. The di- or polyamine compound may be used to replace of from n/10 to n/2 moles of the compound of formula (II) used in the reaction for preparing the prepolymer.

The present invention will now be further explained with reference to specific examples. Dynamic viscosities were measured by using a Bohlin CS50 rheometer at 23°C.

### EXAMPLES

### Example 1

In a 250 ml flask equipped with stirrer, and condenser 29.982 g (279.79 mmol) benzyl amine and 27.730 g (139.89 mmol) butanediol diacrylate were homogeneously mixed and stirred for 3 days at room temperature. Thereafter no double bonds were found in the IR-spectrum at 1609 and 809 cm⁻¹
Yield: 57.71 g (100 % d. Th), h _{23°C} = 0.627 ± 0.011 Pa*s
C₂₄H₃₂N₂O₄; 412.53
IR: 3027, 2954, 2837 (CH₂/CH₃), 1726 (CO), 1453, 1403, 1353, 1170 (CH₂/CH₃) cm⁻¹
¹³C-NMR (CDCl₃): 171.9 (8), 139.6 (2), 128.2-126.5 (3-5), 63.4 (9), 53.2 (1), 44.0 (6), 42.7 (7), 24.8 (10), ppm 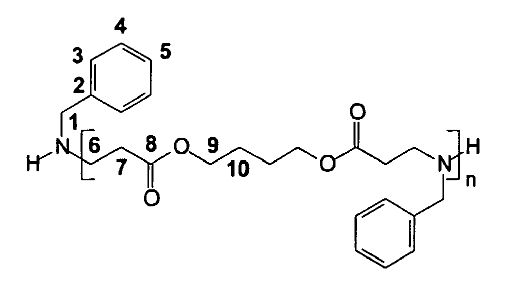 FAB-MS m/z= 413, 718, 1022, 1327, 1633

| | |
|---|---|
| HPLC analysis, content benzyl amine: | 7.17 % (theor. 19.68 %) |
| GC analysis, content benzyl amine: | 6.36 % (theor. 19.68 %). |

### Examples 2-4

Analogous to example 1 benzyl amine BA and butanediol diacrylate BDODA were reacted. The used quantities of monomers, their mol ratio, calculated molecular mass and remaining benzyl amine, calculated and measured by using of GC as well as their viscosities are summarised in Table 1.

**Table 1 Amino terminated prepolymers**

| BA | | BDODA | | mol ratio | Mn (calc) | benzyl amine residue | | Viscosity |
|---|---|---|---|---|---|---|---|---|
| G | mmol | g | mmol | | g/mol | Calc. % | GC % | Pa*s |
| 19.694 | 183.78 | 24.286 | 122.52 | 3/2 | 717.92 | 8.17 | 0.20 | 0.507 ±0.004 |
| 21.241 | 198.22 | 29.468 | 148.66 | 4/3 | 1023.30 | 4.45 | 0.03 | 0.474 ± 0.007 |
| 20.110 | 187.66 | 29.759 | 150.13 | 5/4 | 1328.68 | 2.79 | 0.02 | 0.438 ±0.005 |

### Example 5

In a 250 ml flask equipped with stirrer, and condenser 29.849 g (197.35 mmol) 1-aminoadamantan and 19.559 g (98.67 mmol) butanediol diacrylate were homogeneously mixed and stirred for 5 days at room temperature. Thereafter no double bonds were found in the IR-spectrum at 1609 and 809 cm⁻¹.
Yield: 49.408 g (100 % d. Th), η _{23°C}= 0.835 ± 0.020 Pa*s
C₃₀H₄₈N₂O₄; 500.72
IR: 3303 (OH), 2958, 2924 (CH₂/CH₃), 1725, 1720 (CO), 1458, 1408, 1355, 1170 (CH₂/CH₃) cm⁻¹
¹³C-NMR: 173.1 (7), 64.5/62.0/61.3 (8), 51.7/50.8 (5), 46.2 (1), 42.7 (2), 36.8/36.4/36.1 (4), 35.6/35.5 (6), 29.7/29.3 (9), 25.4 (3) ppm
FAB-MS m/z= 500
GC analysis, content 1-aminoadamantan: 3,23 % (theor. 18.10 %).

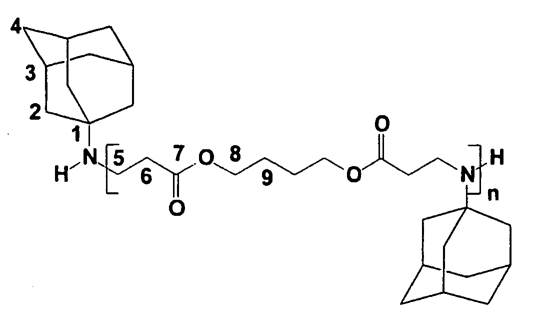

### Example 6

In a 250 ml flask equipped with stirrer, and condenser 17.079 g (279.62 mmol) ethanol amine and 27.713 g (139.81 mmol) butanediol diacrylate were homogeneously mixed and stirred for 5 days at room temperature. Thereafter no double bonds were found in the IR-spectrum at 1609 and 809 cm⁻¹.
Yield: 44.79 g (100 % d. Th), h _{23°C}= 6.257 ± 0.180 Pa*s
C₁₄H₂₈N₂O₆; 320.39
IR: 3303 (OH), 2958, 2924 (CH₂/CH₃), 1725, 1720 (CO), 1458, 1408, 1355, 1170 (CH₂CH₃) cm⁻¹
¹³C-NMR: 173.4/172:9 (5), 64.7/64.2 (6), 62.3/61.7/60.8 (2), 51.3 (1), 44.9 (3), 34.6 (4), 29.9 29.3/25.1/25.3 (7) ppm 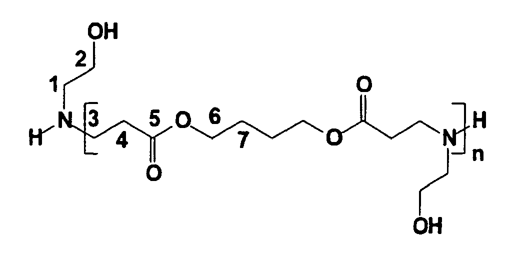 FAB-MS m/z= 321, 579, 839, 1097
GC analysis, content ethanol amine: 0.05 % (theor. 21.66 %).

### Example 7

In a 250 ml flask equipped with stirrer, and condenser 22.857 g (213.30 mmol) benzyl amine and 21.067 g (71.10 mmol) trimethylol propane triacrylate were homogeneously mixed and stirred for 3 days at room temperature. Thereafter no double bonds were found in the IR-spectrum at 1609 and 809 cm⁻¹.
Yield: 43.924 g (100 % d. Th), η _{23°C}= 1.142 ± 0.018 Pa*s
C₃₆H₄₇N₃O₆; 617.79
GC analysis, content benzyl amine: 7.19 %.

### Example 8

25.000 g (233.30 mmol) benzyl amine and 23.592 g (116.65 mmol) 1,4-butandiol diglycidyl ether were homogeneously mixed under heating and polymerised for 24 hours at 50°C.
Yield: 48.592 (100 % of th.)
h _{23°C}= 2.377 ± 0.041 Pa*s
C₂₄H₃₆N₂O₄, 416.56
IR: 3028, 2863, 2860 (CH₂/CH₃), 1451, 1105, 738 (CH₂/CH₃) cm⁻¹
¹³C-NMR: 137.2 (4), 128.3 (2), 128.1 (3), 126.8 (1), 75.2 (8), 71.5 (7), 70.5 (9), 57.8 (5), 55.5 (6), 27.3 (10) ppm (calc.)

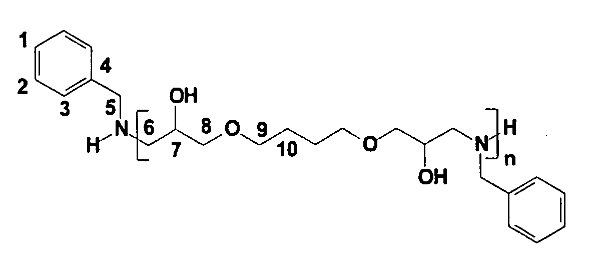

2.784 g (6.683 mmol) of the prepared prepolymer were homogeneously mixed with 3.422 g (6.683 mmol) ethoxylated bisphenol A diacrylate (SR-601, Sartomer) and reacted at 37 °C.

### Example 9

10.000 g (29.375 mmol) bis-2,2-[4-(2,3-epoxypropoxy)-phenyl]-propane and 6.296 g (58.751 mmol) benzyl amine were homogeneously mixed under heating and polymerised for 20 hours at 100°C.
Yield: 16.296 (100 % of th.)
C₃₅H₄₂N₂O₄; 554.73Mₙ(vpO) 620 g/mol, T_{g}11°C

### Example 10

10.000 g (29.375 mmol) bis-2,2-[4-(2,3-epoxypropoxy)-phenyl]-propane and 4.722 g (44.063 mmol) benzyl amine were homogeneously mixed under heating and polymerised for 20 hours at 100°C.
Yield: 14.722 (100 % of th.)
C₆₃H₇₅N₃O₈; 1002.30 g/molMₙ(vpo) 1100 g/mol, T_{g} 30°C

### Application Example 1

1.198 g of amino terminated prepolymer prepared by addition reaction of benzylamine and trimethylol propane triacrylate according of example 7 and 3.133 g of a powder composed of cyclohexane dimethanol diacrylate 16.439 %, calcium tungstate 66.849 % and zirconium oxide 16.712 % were homogeneously mixed and polymerised at 37 °C for 17 hours. The radio-opacity according ISO 6876 is 10.6 mm/mm Al.

### Application Example 2

1.267 g (2.16 mmol) of amino terminated prepolymer prepared by addition reaction of benzylamine and trimethylol propane triacrylate according of example 7 and 3.313 g (2.16 mmol) of a powder composed of cyclohexane dimethanol diacrylate 20.00 % and bismuth oxide 80.00 % were homogeneously mixed and polymerised at 37 ° for 10 hours.
The radio-opacity according ISO 6876 is 8.1 mm/mm Al.

### Application Example 3

| | |
|---|---|
| Acrylate Paste: | 5.000 g (9.77 mmol) ethoxylated bisphenol A diacrylate (SR-601, Sartomer). 9.917 g calcium tungstate, 2.479 g zirconium oxide, 0.050 g arosil A 200 and 0.025 g iron (III) oxide were homogeneously mixed. |
| | |
| Prepolymer Paste: | 4.830 g (9.77 mmol) aminoterminated prepolymer according example 6, 9.500 g calcium tungstate, 2.375 g zirconium oxides and 0.536 g arosil A 200 were homogeneously mixed. |

Immediately before application 1.00 g of the acrylate paste and 0.987 g of the Amino-Prepolymer Paste were mixed homogeneously and polymerised at 37 °C for 3 hours.

### Reference Example

In a 250 ml flask equipped with stirrer and condenser 25.000 g (128.70 mmol) 3,(4),8,(9)-bis(aminomethyl) tricyclo-5.2.1.0^{2.6} decane, 12.755 g (64.35 mmol) butanediol diacrylate were homogeneously mixed and stirred at 20 to 25 °C for 3 days. Thereafter no double bonds were found in the IR-spectrum at 1609 and 809 cm⁻¹. Yield: 37.764 g (100 % d. Th), η 23 °C = 7157 ± 100 Pa*s (after storage for 1 month at room temperature)

2.127 g (3.624 mmol) of the prepared prepolymer were homogeneously mixed with 0.716 g (2.416 mmol) trimethylol propane triacrylate and reacted at 37 °C. The mixture has a gel time of 30 minutes at 37 °C. The prepolymer has a viscosity which is not suitable for the application as a root canal sealing composition.

## Claims

1. A dental root canal sealing composition curable in the absence of a polymerisation initiator and having a viscosity at 23°C of less than 100 Pas, which comprises
(i) an amino terminated prepolymer having a viscosity at 23°C of less than 100 Pas obtainable by reacting
(a) one mole of a compound of the following formula (I) 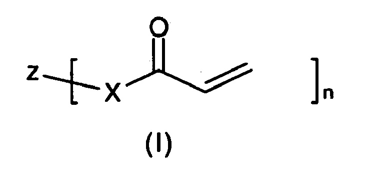 wherein
X represents a nitrogen or an oxygen atom;
Z represents an n-valent saturated aliphatic or cycloaliphatic C₂₋₁₆ hydrocarbon group which may contain 1 to 6 oxygen atoms, and which may be substituted by one or more C₁₋₄ alkyl groups; and
n represents an integer of from 2 to 6; and
(b) at least n moles of one or more compounds either of the following formula (II) 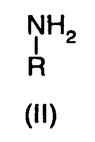
wherein
R represents
a C₁₋₆ alkyl or a C₃₋₁₄ cycloalkyl group, which may be substituted by one or more (up to six) members of the group selected from a C₁₋₄ alkyl group, C₁₋₄alkoxy group, a phenyl group, and a hydroxy group,or
of formula (II) in combination with one or more di- or polyamine compound.
(ii) a di-or polyfunctional acrylate compound or a di- or polyfunctional maleimide compound which is capable of undergoing polyaddition with the aminoterminated prepolymer (i);
(iii) 40 to 85 wt.-% of a filler for providing a minimum radioopacity of at least 3mm/mm Al;
said compositon being in the form of a two-component composition wherein a first component contains the amino terminated prepolymer (i) and
optionally filler (iii) and a second component contains the compound (ii) capable of undergoing polyaddition with the aminoterminated prepolymer (i) and optionally filler (iii).

2. The dental root canal sealing composition according to claim 1 wherein the composition contains a prepolymer of the following formula (III) 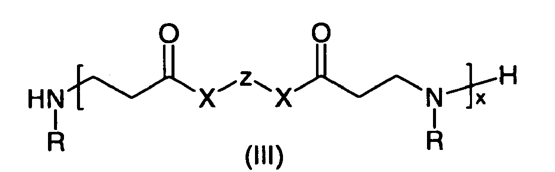 wherein
X represents an oxygen or nitrogen atom;
Z represents an n-valent saturated aliphatic C₂₋₁₆ hydrocarbon group or a divalent saturated cycloaliphatic C₃₋₆ hydrocarbon group, which groups may contain 1 to 6 oxygen atoms, and which may be substituted by 1 to 6 C₁₋₄ alkyl groups; and
R represents a C₁₋₄ alkyl or a C₃₋₁₄ cycloalkyl group, which may be substituted by a C₁₋₄ alkyl group, a phenyl group, or a hydroxy group; and
x is an integer of from 1 to 8;
(ii) a di-or polyfunctional acrylate compound or a di- or polyfunctional maleimide compound which is capable of undergoing polyaddition with the aminoterminated prepolymer (i);
(iii) 40 to 85 wt.-% of a filler for providing a minimum radioopacity of at least 3mm/mm AI.

3. The dental root canal sealing composition according to any one of the preceding claims, wherein
Z represents a divalent saturated aliphatic C₂₋₁₀ hydrocarbon chain, which may be substituted by 1 to 6 C₁₋₄alkyl groups.

4. The dental root canal sealing composition according to any one of the preceding claims, wherein Z represents a C₂₋₆ alkylene group.

5. The dental root canal sealing composition according to any one of the preceding claims, wherein X represents an oxygen atom.

6. The dental root canal sealing composition according to claims 1-4, wherein X represents a nitrogen atom.

7. The dental root canal sealing composition according to any one of the preceding claims, wherein R is selected from a C₁₋₄alkyl group, which may be substituted by a phenyl group, a hydroxy group, or an amino group.

8. The dental root canal sealing composition according to any one of the preceding claims, wherein the filler contains La₂O₃, ZrO₂, BiPO₄, CaWO₄, BaWO₄, SrF₂, Bi₂O₃.

9. The dental root canal sealing composition according to any one of the preceding claims, wherein the two-component composition is a powder/liquid or a paste/paste system.

10. The dental root canal sealing composition according to any one of the preceding claims, wherein the amino terminated prepolymer is obtainable by reacting
(i) one mole of a compound of the following formula (IV) 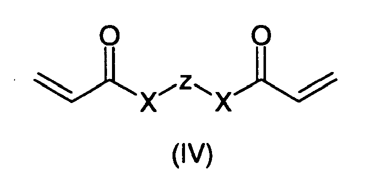 wherein X and Z are as defined as in claim 2, and
(ii) at least two moles of one or more compounds of the following formula (II) 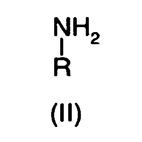 wherein R represents a C₁₋₄ alkyl or a C₃₋₁₄ cycloalkyl group, which may be substituted by a C₁₄ alkyl group, a phenyl group, or a hydroxy group.

## Patentansprüche

1. Dentale Wurzelkanal-Versiegelungszusammensetzung, die in Abwesenheit eines Polymerisationsinitiators härtbar ist, bei 23°C eine Viskosität von weniger als 100 Pa*s aufweist, und die umfasst:
(i) ein Amino-terminiertes Präpolymer mit einer Viskosität bei 23°C von weniger als 100 Pa*s, das erhältlich ist durch Umsetzung
(a) von einem Mol einer Verbindung der folgenden Formel (I) 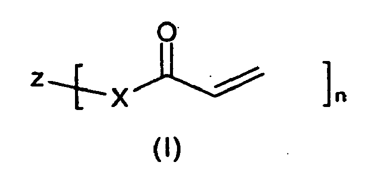 wobei
X ein Stickstoff- oder ein Sauerstoffatom bedeutet;
Z für eine n-wertige gesättigte aliphatische oder cycloaliphatische C₂₋₁₆ Kohlenwasserstoffgruppe, die 1-6 Sauerstoffatome aufweisen kann, steht, und die substituiert sein kann mit einer oder mehreren C₁₋₄ Alkylgruppen; und
n für eine ganze Zahl von 2 bis 6 steht; und
(b) mindestens n Mol einer oder mehrerer Verbindungen, entweder der folgenden Formel (II) 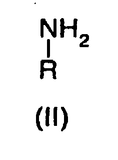
wobei
R für eine C₁₋₆ Alkyl- oder C₃₋₁₄ Cycloalkylgruppe steht, die substituiert sein kann mit einem oder mehreren (bis zu 6) Elementen der Gruppe, ausgewählt aus einer C₁₋₄ Alkylgruppe, einer C₁₋₄ Alkoxygruppe, einer Phenylgruppe, und einer Hydroxygruppe,
oder der Formel (II) in Kombination mit einer oder mehreren Di- oder Polyaminverbindungen,
(ii) eine di- oder polyfunktionelle Acrylatverbindung oder eine di- oder polyfunktionelle Maleimidverbindung, die geeignet ist eine Polyaddition mit dem Amino-terminierten Präpolymer (i) einzugehen;
(iii) 40 bis 85 Gew.-% eines Füllstoffs zur Bereitstellung einer Mindestradioopazität von mindestens 3 mm/mm Al;
wobei die Zusammensetzung in Form einer Zwei-Komponenten-Zusammensetzung vorliegt, wobei eine erste Komponente das Amino-terminierte Präpolymer (i) und gegebenenfalls Füllstoff (iii) enthält und eine zweite Komponente die Verbindung (ii) enthält, die geeignet ist eine Polyaddition mit dem Amino-terminierten Präplolymer (i) einzugehen, sowie gegebenenfalls Füllstoff (iii).

2. Die dentale Wurzelkanal-Versiegelungszusammensetzung gemäß Anspruch 1, wobei die Zusammensetzung ein Präpolymer der folgenden Formel (III) enthält 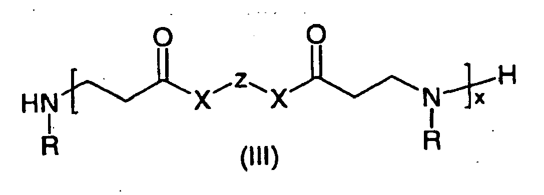 wobei
X für ein Sauerstoff- oder Stickstoffatom steht;
Z eine n-wertige gesättigte aliphatische C₂₋₁₆ Kohlenwasserstoffgruppe oder eine divalente gesättigte cycloaliphatische C₃₋₆ Kohlenwasserstoffgruppe bedeutet, wobei diese Gruppen 1 bis 6 Sauerstoffatome enthalten können und mit 1 bis 6 C₁₋₄ Alkylgruppen substituiert sein können; und
R für eine C₁₋₄ Alkyl- oder eine C₃₋₁₄ Cycloalkylgruppe steht, die substituiert sein kann mit einer C₁₋₄ Alkylgruppe, einer Phenylgruppe oder einer Hydroxygruppe; und
x für eine ganze Zahl von 1 bis 8 steht;
(ii) eine di- oder polyfunktionelle Acrylatverbindung oder eine di- oder polyfunktionelle Maleimid-Verbindung, die geeignet ist eine Polyaddition mit dem Amino-terminierten Präpolymer (i) einzugehen;
(iii) 40 bis 85 Gew.-% eines Füllstoffs zur Bereitstellung einer Minimumradioopazität von mindestens 3 mm/mm AI.

3. Die dentale Wurzelkanal-Versiegelungszusammensetzung gemäß einem der vorstehenden Ansprüche, wobei
Z für eine di-valente gesättigte aliphatische C₂₋₁₀ Kohlenwasserstoffkette steht, die substituiert sein kann mit 1 bis 6 C₁₋₄ Alkylgruppen.

4. Die dentale Wurzelkanal-Versiegelungszusammensetzung gemäß einem der vorstehenden Ansprüche, wobei Z für eine C₂₋₆Alkylengruppe steht.

5. Die dentale Wurzelkanal-Versiegelungszusammensetzung gemäß einem der vorstehenden Ansprüche, wobei X für ein Sauerstoffatom steht.

6. Die dentale Wurzelkanal-Versiegelungszusammensetzung gemäß einem der Ansprüche 1-4, wobei X für ein Stickstoffatom steht.

7. Die dentale Wurzelkanal-Versiegelungszusammensetzung gemäß einem der vorstehenden Ansprüche, wobei R ausgewählt wird aus einer C₁₋₄ Alkylgruppe, die substituiert sein kann mit einer Phenylgruppe, einer Hydroxygruppe oder einer Aminogruppe.

8. Die dentale Wurzelkanal-Versiegelungszusammensetzung gemäß einem der vorstehenden Ansprüche, wobei der Füllstoff La₂O₃, ZrO₂, BiPO₄, CaWO₄, BaWO₄, SrF₂, oder Bi₂O₃ enthält.

9. Die dentale Wurzelkanal-Versiegelungszusammensetzung gemäß einem der vorstehenden Ansprüche, wobei die Zwei-Komponenten-Zusammensetzung ein Pulver/Flüssigkeit- oder ein Paste/Paste-System ist.

10. Die dentale Wurzelkanal-Versiegelungszusammensetzung gemäß einem der vorstehenden Ansprüche, wobei das Amino-terminierte Präpolymer erhältlich ist durch Reaktion von
(i) einem Mol einer Verbindung der folgenden Formel (IV) 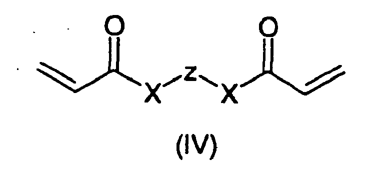 wobei X und Z die in Anspruch 2 definierten Bedeutungen haben, und
(ii) mindestens zwei Mol von einer oder mehreren Verbindungen der folgenden Formel (II) 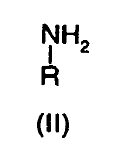 wobei R für eine C₁₋₄ Alkyl- oder eine C₃₋₁₄ Cycloalkylgruppe steht,
die substituiert sein kann mit einer C₁₋₄ Alkylgruppe, einer Phenylgruppe, oder einer Hydroxygruppe.

## Revendications

1. Composition d'obturation de canal radiculaire dentaire, durcissable en l'absence d'initiateur de polymérisation et ayant une viscosité à 23°C inférieure à 100 Pas, qui comprend
(i) un prépolymère à terminaison amino ayant une viscosité à 23°C inférieure à 100 Pas pouvant être obtenu en faisant réagir
(a) une mole d'un composé répondant à la formule (I) suivante 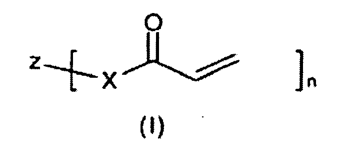 dans laquelle
X représente un atome d'azote ou un atome d'oxygène
Z représente un groupe hydrocarboné en C₂ à C₁₆ aliphatique ou cycloaliphatique saturé de valence n qui peut contenir 1 à 6 atomes d'oxygène, et qui peut être substitué avec un ou plusieurs groupes alkyle en C₁ à C₄ ; et
n représente un nombre entier de 2 à 6 ; et
(b) au moins n moles d'un ou plusieurs composés soit de la formule (II) suivante 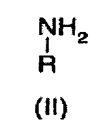 dans laquelle
R représente
un groupe alkyle en C₁ à C₆ ou cycloalkyle en C₃ à C₁₄, qui peut être substitué avec un ou plusieurs (jusqu'à 6) membres du groupe choisi entre un groupe alkyle en C₁ à C₄, un groupe alkoxy en C₁ à C₄, un groupe phényle et un groupe hydroxy, soit
de formule (II) en association avec un ou plusieurs composés du type di- ou polyamine ;
(ii) un composé du type acrylate di- ou polyfonctionnel ou un composé du type maléimide di- ou polyfonctionnel qui est capable de subir une polyaddition avec le prépolymère à terminaison amino (i) :
(iii) 40 à 85 % en poids d'une charge pour parvenir à une radio-opacité minimale d'au moins 3 mm/mm de A1 ;
ladite composition étant sous forme d'une composition à deux constituants dans laquelle un premier constituant contient le prépolymère à terminaison amino (i) et facultativement une charge (iii) et un second constituant contient le composé (ii) capable de subir une polyaddition avec le prépolymère à terminaison amino (i) et facultativement la charge (iii).

2. Composition d'obturation de canal radiculaire dentaire suivant la revendication 1, qui contient un prépolymère répondant à la formule (III) suivante 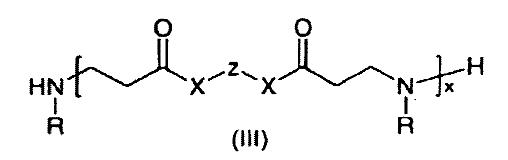 dans laquelle
X représente un atome d'oxygène ou un atome d'azote ;
Z représente un groupe hydrocarboné en C₂ à C₁₆ aliphatique saturé de valence n ou un groupe hydrocarboné en C₃ à C₆ cycloaliphatique saturé divalent, groupes qui peuvent contenir 1 à 6 atomes d'oxygène et qui peuvent être substitués avec un à six groupes alkyle en C₁ à C₄ ; et
R représente un groupe alkyle en C₁ à C₄ ou cycloalkyle en C₃ à C₁₄, qui peut être substitué avec un groupe alkyle en C₁ à C₄, un groupe phényle ou un groupe hydroxy ; et
x représente un nombre entier de 1 à 8 ;
(ii) un composé du type acrylate di- ou polyfonctionnel ou un composé du type maléimide di- ou polyfonctionnel qui est capable de subir une polyaddition avec le prépolymère à terminaison amino (i) :
(iii) 40 à 85 % en poids d'une charge pour parvenir à une radio-opacité minimale d'au moins 3 mm/mm de Al.

3. Composition d'obturation de canal radiculaire dentaire suivant l'une quelconque des revendications précédentes, dans laquelle
Z représente une chaîne hydrocarbonée en C₂ à C₁₀ aliphatique saturée divalente qui peut être substituée avec un à six groupes alkyle en C₁ à C₄.

4. Composition d'obturation de canal radiculaire dentaire suivant l'une quelconque des revendications précédentes, dans laquelle Z représente un groupe alkylène en C₂ à C₆.

5. Composition d'obturation de canal radiculaire dentaire suivant l'une quelconque des revendications précédentes, dans laquelle X représente un atome d'oxygène.

6. Composition d'obturation de canal radiculaire dentaire suivant les revendications 1 à 4, dans lesquelles X représente un atome d'azote.

7. Composition d'obturation de canal radiculaire dentaire suivant l'une quelconque des revendications précédentes, dans laquelle R est choisi parmi des groupes alkyle en C₁ à C₄, qui peuvent être substitués avec un groupe phényle, un groupe hydroxy ou un groupe amino.

8. Composition d'obturation de canal radiculaire dentaire suivant l'une quelconque des revendications précédentes, dans lesquelles la charge contient La₂O₃, ZrO₂, BiPO₄, CaWO₄, BaWO₄, SrF₂, Bi₂O₃.

9. Composition d'obturation de canal radiculaire dentaire suivant l'une quelconque des revendications précédentes, ladite composition à deux constituants étant un système de poudre / liquide ou un système de pâte / pâte.

10. Composition d'obturation de canal radiculaire dentaire suivant l'une quelconque des revendications précédentes, dans laquelle le prépolymère à terminaison amino peut être obtenu en faisant réagir
(i) une mole d'un composé répondant à la formule (IV) suivante 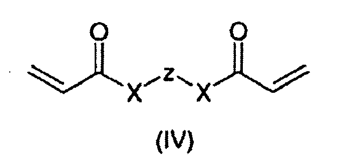 dans laquelle X et Z sont tels que définis dans la revendication 2, et
(ii) au moins deux moles d'un ou plusieurs composés répondant à la formule (II) suivante 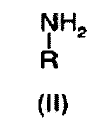 dans laquelle R représente un groupe alkyle en C₁ à C₄ ou un groupe cycloalkyle en C₃ à C₁₄, qui peut être substitué avec un groupe alkyle en C₁ à C₄, un groupe phényle ou un groupe hydroxy.
